# EUROPÄISCHE PATENTANMELDUNG

(11) **EP 0 693 287 A1**
(43) Veröffentlichungstag der Anmeldung: **24.01.1996**
(21) Anmeldenummer: 95111064.2
(22) Anmeldetag: 14.07.1995
(51) Int. Cl.: A61K 48/00, C12N 15/63, C12N 15/11, C07H 21/00

(54) **RNA-spaltende bzw. RNA-bindende Oligonucleotide**

(30) Priorität: 18.07.1994 DE 4425311
(71) Anmelder: HOECHST AKTIENGESELLSCHAFT, D-65929 Frankfurt am Main (DE)
(72) Erfinder: Peyman, Anuschirwan, Dr., D-65779 Kelkheim (DE); Uhlmann, Eugen, Dr., D-61479 Glashütten (DE)

(57) **Zusammenfassung**

Es werden therapeutische Mittel zur Inhibierung der Protein-Synthese enthaltend zwei Antisense-Oligonucleotide AO-1 und AO-2 mit ca. 6 - 100 Nucleotiden, die an benachbarter Stelle an der Ziel-RNA binden und am 5'-Ende bzw. 3'-Ende über geeignete Spacer mit Konjugatmolekülen A und B verknüpft sind, wobei die Konjugatmoleküle A und B jedes für sich nicht reaktiv ist, aber in räumliche Nähe gebracht, eine Einheit bilden, und so die RNA katalytisch zu spalten vermag oder durch die räumliche Nähe aktiviert zur kovalenten Bindung von RNA führt, beschrieben.

## Beschreibung

Die Erfindung betrifft therapeutische Mittel zur Inhibierung der Protein-Synthese, enthaltend zwei Antisense-Oligonucleotide, die an benachbarter Stelle an der Ziel-RNA binden und am 5'-Ende bzw. 3'-Ende über geeignete Spacer mit Konjugatmolekülen verknüpft sind.

Antisense-Oligonucleotide werden zur Inhibierung der Protein-Synthese breit eingesetzt (siehe beispielsweise E. Uhlmann, A. Peyman, Chem. Rev. 90 (1990) 543). Ihre Wirkungsweise beruht auf der Hybridisierung an die komplementäre Sequenz der Pre-mRNA oder der RNA, welche für das zu inhibierende Protein kodiert. Als Folge dieser Hybrid-Bildung wird die Protein-Biosynthese dieses Proteins unterbunden, wobei verschiedene Mechanismen zum Tragen kommen können, wie beispielsweise Transkriptionsblockierung, Inhibierung des Splice Prozesses oder der mRNA Reifung oder aber der Abbau der mRNA durch RNase H. Letzterer wird jedoch nur mit einigen wenigen chemischen Varianten erreicht, die zur Stabilisierung der Antisense Oligonucleotide gegen Nuclease-Abbau notwendig sind. Einen Überblick über den Stand der Technik geben beispielsweise J. F. Milligan, M. D. Matteucci und J. C. Martin, J. Med. Chem. 36 (1993) 1923 sowie S. T. Crooke, Annu. Rev. Pharmacol. Toxicol. 32 (1992) 329.

Eine andere Möglichkeit, RNA zu zerstören und so die Protein-Biosynthese zu unterbinden geben chemische Nucleasen. Es existieren eine ganze Reihe chemischer Nucleasen, die in der Lage sind, RNA zu spalten (siehe D. S. Sigman et al., Chem. Rev. 93 (1993) 2295; D. S. Sigman et al., Acc. Chem. Res. 26 (1992) 98):
1) Beispielsweise wird ein Modell für das RNA spaltende Enzym Ribonuclease A, beschrieben (siehe auch Breslow, Acc. Chem. Res. 24 (1991) 317), das lediglich die räumliche Nähe zweier Imidazol-Derivate erfordert, die durch Konjugation an Cyclodextrin-Gerüsten erreicht wird. Ein Imidazol an Cyclodextrin allein ist sehr viel weniger reaktiv.
2) Ein anderes Ribonuclease-Modell wird von J. Smith et al. (J. Am. Chem. Soc. 115 (1993) 362) beschrieben, dem die stark beschleunigte Spaltung von RNA durch Imidazol in Gegenwart der Verbindung der Formel A gelang. 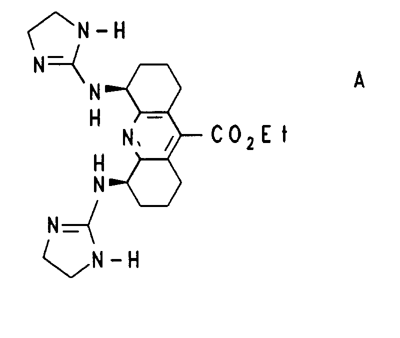
3) Ein weiteres RNase Modell wird erläutert, worin Moleküle der Formel B oder C, Zink-Komplexe von 1,10-Phenanthrolin-2-carbinol oder N-dodecyl-2-(aminomethyl)-1,10-phenanthrolin verwendet werden (D. S. Sigman et al., Chem. Rev. 93 (1993) 2295). 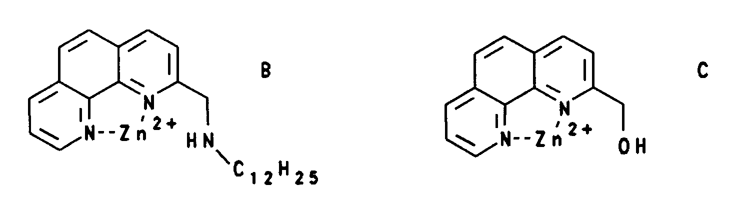
4) Tripeptide wie L-Lysyl-L-Tryptophanyl-L-Lysin, die in der Lage sind, DNA über einen Eliminierungsmechanismus zu spalten, werden von D. S. Sigman et al. (Chem. Rev. 93 (1993) 2295) beschrieben.
5) Eine Vielzahl weiterer Metallkomplexe sind beschrieben, die in der Lage sind, RNA bzw. DNA oxidativ zu spalten, beispielsweise einen 2:1 Komplex von 1,10-Phenanthrolin (OP) und Kupfer zur RNA-Spaltung oder oktaedrische (OP)₃Ru²⁺-Komplexe oder Metall-chelierende Tripeptide, wie beispielsweise Glycyl-Glycyl-L-Histidin Komplexe mit Cu(II) oder Ni(II) oder Glycyl-L-Histidyl-L-Lysin-Komplexe mit Cu(II) (D. S. Sigman et al., Chem. Rev. 93 (1993) 2295).

Nachteil dieser chemischen Nucleasen ist, daß sie DNA bzw. RNA-Sequenzen nicht spezifisch erkennen können und daher äußerst unselektiv sind.

Es gibt nun verschiedene Ansätze zur Wirkungssteigerung von Antisense-Oligonucleotiden (J. Goodchild, Bioconjugate Chem. 1 (1990) 165), die darauf beruhen, daß der Komplex mit der RNA stabilisiert wird, beispielsweise durch Konjugation der Oligonucleotide mit Crosslinkern wie beispielsweise Psoralen oder Interkalatoren wie beispielsweise Acridin. Andere Ansätze beinhalten die Verknüpfung von Antisense-Oligonucleotiden mit chemischen Nucleasen. Dafür werden Oligonucleotide mit Molekülen verknüpft, die in der Lage sind, RNA zu spalten, beispielsweise mit Metallkomplexen wie EDTA-Fe(II) oder o-Phenanthrolin-Cu(I) oder Porphyrin-Fe(II) (E. Uhlmann and A. Peyman, Chemical Reviews 90 (1990) 543) oder Terpyridin-Cu(II), Bipyridin-Cu(II) und andere, wie sie in WO 91/19730 beschrieben sind, oder Oligonucleotid-Tripeptid-Konjugate, wie sie in WO 93/02689 beschrieben sind. Auch Konjugate von Oligonucleotiden mit Enzymen wie beispielsweise Staphylokokken-Nuclease sind beschrieben (E. Uhlmann and A. Peyman, Chemical Reviews 90 (1990) 543). J. K. Bashkin et al. (J. Org. Chem. 55 (1990) 5125) und N. N. Polushin et al. (J. Org. Chem. 58 (1993) 4606) beschreiben die Synthese von Nucleosid-Monomer-Imidazol-Konjugaten, mit dem Ziel, diese in Oligonucleotide einzubauen und die RNA-spaltende Aktivität von Imidazol und die spezifische Bindung von Oligonucleotiden zu kombinieren.

Ein großer Nachteil dieser Technologie ist die mangelnde Spezifität solcher Konjugate. Es besteht die Gefahr, daß Crosslinking bzw. RNA-Spaltung eintritt noch bevor das Oligonucleotid spezifisch an die Ziel-RNA gebunden hat. Webb und Matteucci (Nucl. Acids Res. 14 (1986) 7661; J. Am. Chem. Soc. 108 (1986) 2764) führten daher den Begriff des "hybridization triggered crosslinking" ein, sie verwendeten ein 5-Methyl-N⁴,N⁴-ethanocytosin enthaltendes Oligonucleotid, welches erst nach erfolgter Hybridisierung in der Lage ist, mit der Ziel-RNA zu reagieren.
Für die RNA-Spaltung, möglichst unter physiologischen Bedingungen ohne Zuhilfenahme von Hilfsreagentien oder Bestrahlung, gibt es ein solches Konzept nicht.

Ziel dieser Arbeit ist es daher, die Selektivität bzw. Spezifität solcher chemischen Nucleasen zu verbessern.

Gegenstand der Erfindung sind daher therapeutische Mittel zur Inhibierung der Protein-Synthese enthaltend zwei Antisense-Oligonucleotide AO-1 und AO-2 mit ca. 6 - 100, vorzugsweise ca. 10 - 40, insbesondere ca. 12 - 25 Nucleotiden, die an benachbarter Stelle an der Ziel-RNA binden und am 5'-Ende bzw. 3'-Ende über geeignete Spacer mit Konjugatmolekülen A und B verknüpft sind (erhalten durch Spaltung von chemischen Nucleasen), wobei die Konjugatmoleküle A und B jedes für sich nicht reaktiv ist, aber in räumliche Nähe gebracht, eine Einheit bilden, die RNA katalytisch zu spalten vermag oder durch die räumliche Nähe aktiviert zur kovalenten Bindung von RNA führt.

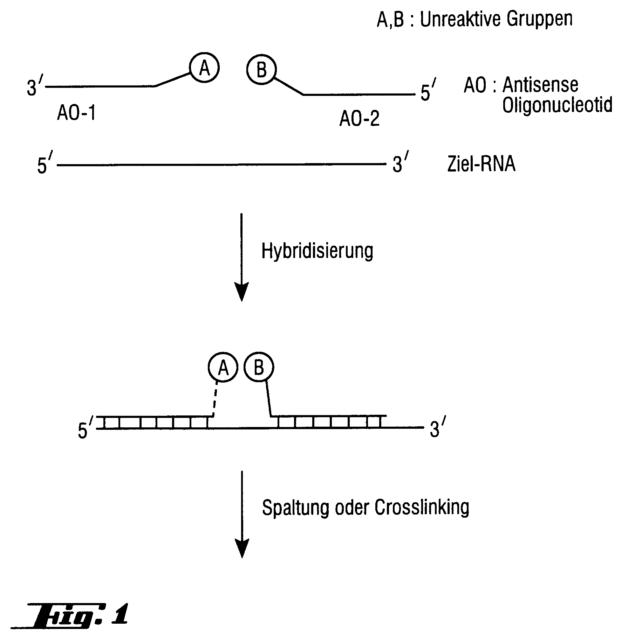

Wie Fig 1 zeigt, wird eine reaktive Einheit von A und B also erst gebildet, wenn beide Antisense-Oligonucleotide spezifisch an den benachbarten Stellen der Ziel-RNA gebunden sind. Crosslinking bzw. RNA-Spaltung tritt erst ein, nachdem die Antisense-Oligonucleotide spezifisch an die Ziel-RNA gebunden haben. Die Einführung einer reaktiven Gruppierung ist somit an einen erheblichen Spezifitätsgewinn verknüpft und nicht wie sonst üblich mit einem Verlust an Spezifität.

Bevorzugt werden Antisense-Oligonucleotide AO-1 und AO-2 mit nachfolgenden Konjugatmoleküle A und B eingesetzt, die mit dem Antisense-Oligonucleotid über einen geeigneten Spacer verknüpft sind:
a) A = B = Imidazol;
b) A = Imidazol, B = Molekül der Formel I 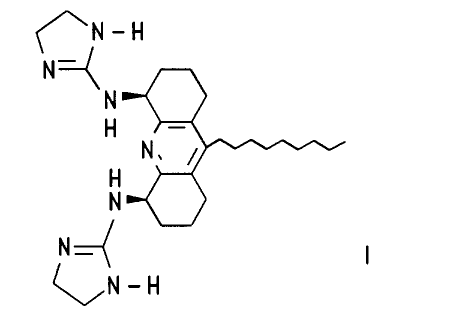
c) A = B = 1,10-Phenanthrolin;
d) A = L-Lysin, B = L-Tryptophanyl-L-Lysin;
e) A = L-Lysyl-L-Tryptophan, B = L-Lysin;
f) A = Glycin, B = Glycyl-L-Histidin;
g) A = Glycyl-Glycin, B = L-Histidin;
h) A = Glycin, B = L-Histidyl-L-Lysin;
i) A = Glycyl-L-Histidin, B = L-Lysin;
wobei die Konjugatmoleküle aus c) und f) - i) jeweils mit dem Oligonucleotid über einen geeigneten Spacer zur Bildung eines Cu(II)-Komplexes verknüpft sind.

Besonders bevorzugt sind Konjugatmoleküle A = B = Imidazol.

Die Antisense-Oligonucleotide können unmodifiziert oder modifiziert vorliegen, wobei die dem Fachmann bekannten Modifikationen zum Einsatz kommen können. Insbesondere sind darunter chemische Modifikationen zu verstehen, die die Eigenschaften von Oligonucleotiden, wie beispielsweise Nuclease-Stabilität und Zellgängigkeit, verbessern, und die dem Fachmann bekannt sind (siehe beispielsweise: E. Uhlmann und A. Peyman, Chem. Rev. 90 (1990) 543; P. D. Cook, Anti-Cancer Drug Design 6 (1991) 585); J. Goodchild, Bioconjugate Chem. 1 (1990) 165; S. L. Beaucage und R. P. Iyer, Tetrahedron 49 (1993) 6123; F. Eckstein, Ed., "Oligonucleotides and Analogues - A Practical Approach", IRL Press 1991.)

### Solche Modifikationen sind beispielsweise

a) Modifikationen der Phosphatbrücke, beispielsweise Modifikationen wie Phosphorothioate, Phosphorodithioate, Methylphosphonate, Phosphoramidate, Boranophosphate, Phosphatmethylester, Phosphatethylester, Phenylphosphonate, bevorzugt Phosphorothioate und Methylphosphonate, wobei der Ersatz aller Phosphatbrücken der Antisense-Oligonucleotide durch die genannten Modifikationen ausgenommen sein soll;
b) Ersatz der Phosphatbrücke, beispielsweise durch Formacetal, 3'-Thioformacetal, Methylhydroxylamin, Oxim, Methylendimethylhydrazo, Dimethylensulfon, Silylgruppen, bevorzugt durch Formacetal und 3'-Thioformacetal;
c) Modifikationen des Zuckers, beispielsweise Modifikationen wie α-anomere Zucker, 2'-O-Methylribose, 2'-O-Butylribose, 2'-O-Allylribose, 2'-Fluoro-2'-desoxyribose, 2'-Amino-2'-desoxyribose, α-Arabinofuranose, carbocyclische Zuckeranaloge, bevorzugt 2'-O-Methylribose;
d) Modifikationen der Basen, welche die Spezifität der Watson-Crick Basenpaarung nicht verändern, beispielsweise Modifkationen wie 5-Propin-2'-desoxyuridin, 5-Propin-2'-desoxycytidin, 5-Fluoro-2'-desoxycytidin, 5-Fluor-2'-desoxyuridin, 5-Hydroxymethyl-2'-desoxyuridin, 5-Methyl-2'-desoxycytidin, 5-Brom-2'-desoxycytidin, bevorzugt 5-Propin-2'-desoxyuridin und 5-Propin-2'-desoxycytidin;
e) Ersatz des Zuckerphosphat-Rückgrats, beispielsweise durch "Morpholinonucleosid"-Oligomere (E. P. Stirchak et al., Nucleic Acids Res. 17 (1989) 6129) oder durch "Peptide Nucleic Acids" (z.B. Hanvey et al., Science 258 (1992) 1481);
f) 5'- und 3'-Phosphate, sowie 5'- und 3'-Thiophosphate;
g) Konjugate, beispielsweise am 3'- oder am 5'-Ende (siehe auch DE-P 44 20 737.9 (HOE 94/F 161) zur 3'-Derivatisierung), wie beispielsweise Konjugate mit Poly-Lysin, mit Interkalatoren wie Pyren, Acridin, Phenazin und Phenanthridin, mit fluoreszierenden Verbindungen wie Fluorescein, mit Cross-Linkern wie Psoralen und Azidoproflavin, mit lipophilen Molekülen wie (C₁₂-C₂₀)-Alkyl, mit Lipiden wie 1,2-Di-hexadecyl-rac-glycerin, mit Steroiden wie Cholesterin und Testosteron, mit Vitaminen wie Vitamin E, mit Poly- bzw. Oligoethylengylcol, mit (C₁₂-C₁₈)-Alkyl-Phosphatdiestern, mit -O-CH₂-CH(OH)-O-(C₁₂-C₁₈)-Alkyl;
h) 3'-3'- und 5'-5'-Inversionen (z.B. M. Koga et al., J. Org. Chem. 56 (1991) 3757).

Bevorzugt sind die folgenden Modifikationen:
a) Ersatz der Phosphatbrücken durch Phosphorothioate, Phosphorodithioate oder Methylphosphonate, wobei der Ersatz aller Phosphatbrücken der Antisense-Oligonucleotide durch die genannten Modifikationen ausgenommen sein soll, und
b) Konjugate mit lipophilen Molekülen wie (C₁₂-C₂₀)-Alkyl, mit Steroiden wie Cholesterin und Testosteron, mit Poly- oder Oligoethylenglykol, mit Vitamin E, mit Interkalatoren wie Pyren, mit (C₁₄-C₁₈)-Alkyl-Phosphatdiestern und mit -O-CH₂-CH(OH)-O-(C₁₂-C₁₆)-Alkyl.

Besonders bevorzugte Modifikationen sind:
a) Der Ersatz von ein, zwei oder drei Phosphatbrücken am 5'- und/oder 3'-Ende durch Phosphorothioate und
b) der Ersatz von ein, zwei oder drei Phosphatbrücken am 5'- und/oder 3'-Ende durch Phosphorothioate und zusätzlich der Ersatz von 1 - 5 Phosphatbrücken durch Phosphorothioate in der Mitte.

Gegenstand der Erfindung ist weiterhin ein Verfahren zur Spaltung von RNA bzw. Crosslinking, das durch gekennzeichnet ist, daß man zwei Antisense-Oligonucleotide AO-1 und AO-2 mit ca. 6 - 100, vorzugsweise ca. 10 - 40, insbesondere ca. 12 - 25 Nucleotiden, die an benachbarter Stelle an der Ziel-RNA binden und am 5'-Ende bzw. 3'-Ende über geeignete Spacer mit Konjugatmolekülen A und B verknüpft sind, wobei die Konjugatmoleküle A und B jedes für sich nicht reaktiv ist, in räumliche Nähe bringt, so daß diese eine Einheit bilden und die RNA katalytisch spalten oder aktiviert werden und so zur kovalenten Bindung von RNA führen.

Gegenstand der Erfindung sind ferner Antisense-Oligonucleotide AO-1 und AO-2, insbesondere 3'- und 5'- mit Konjugatmolekülen A und B, wie oben beschrieben, verknüpfte Oligonucleotide, besonders bevorzugt solche 3' und 5' mit Imidazol verknüpfte Oligonucleotide der Formeln II und III. 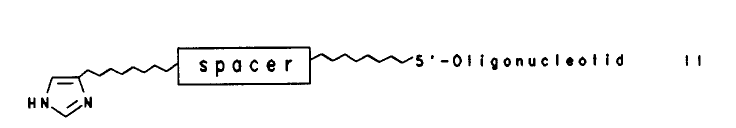 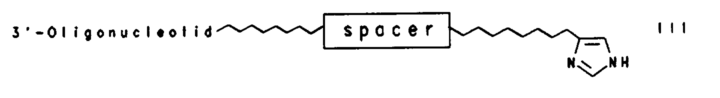
Gegenstand der Erfindung sind weiterhin Verfahren zur Herstellung der Antisense-Oligonucleotide AO-1 und AO-2 nach dem Fachmann bekannten Verfahren (z.B. E. Uhlmann und A. Peyman, Chem. Rev. 90 (1990) 543; "Antisense Research and Applications", Crooke and Lebleu, Eds., CRC Press, Boca Raton, 1993), das dadurch gekennzeichnet ist, daß man die oben beschriebenen Konjugatmoleküle A und B über einen geeigneten Spacer mit Oligonucleotiden verknüpft.

Länge und chemische Struktur der Spacer können stark variieren. Der Spacer kann beispielsweise stehen für (C₁-C₁₈)-Alkyl, welches gegebenenfalls ein oder mehrfach durch Amino, (C₁-C₄)-Alkylamino, Di-(C₁-C₄)-alkylamino, Hydroxy, Oxy, (C₁-C₄)-Alkoxy, Halogen, z.B. Fluor, Chlor oder Brom, Cyano, Carboxy, (C₆-C₁₂)-Aryl, (C₆-C₁₂)-Heteroaryl, mit Heteroatomen aus der Reihe O, N, S, die jeweils 1 - 8 mal vorkommen können, substituiert und gegebenenfalls ein oder mehrfach ungesättigt, verzweigt oder unverzweigt sein kann. Ein oder mehrere Atome der Alkylkette können auch durch Heteroatome aus der Reihe O, N, S ersetzt sein, und der Spacer kann zusätzlich ein bis drei Phosphat-, Thiophosphat-, Dithiophosphat-, Carbonsäureamid-, Carbonsäureester-, Aryl oder Heteroaryl-Brücken enthalten.

Bevorzugt steht der Spacer für (C₁-C₁₈)-Alkyl, wobei ein bis sechs Atome der Alkylkette durch O oder N ersetzt sein können und der Spacer zusätzlich eine Phosphatbrücke oder eine Carbonsäureamidbrücke enthalten kann.

Beispiele für Verbindungen der Formeln II und III sind Verbindungen der Formeln IV und V 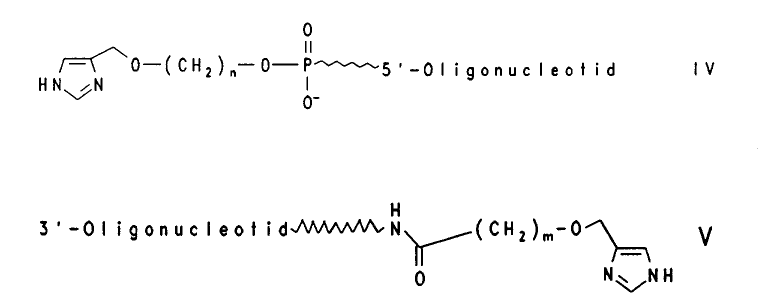
worin
n und m Werte zwischen 2 und 18 bedeuten, besonders bevorzugt Werte zwischen 3 und 8.

Antisense-Oligonucleotide der Formel IV werden dadurch erhalten, daß man zunächst ein Oligonucleotid nach bekannten Verfahren synthetisiert, die 5'-Schutzgruppe, in der Regel die Dimethoxytritylgruppe, abspaltet, dann mit einer Verbindung der Formel VI 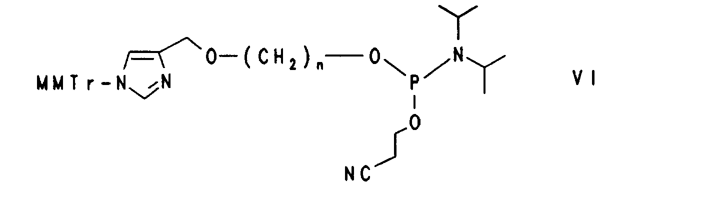
worin
MMTr für Monomethoxytrityl steht,
nach den in der Oligonucleotid-Chemie üblichen Verfahren (S. Agrawal, Ed. Protocols for Oligonucleotides and Analogs, Methods in Molecular Biology, Vol. 20, Humana Press 1993) umsetzt, und anschließend die Schutzgruppen nach bekannten Verfahren abspaltet, beispielsweise durch Behandlung mit Dichloressigsäure und wäßrigem NH₃. Alternativ können auch analoge Derivate von VI über die H-Phosphonat-Methode oder nach dem Triester-Verfahren eingeführt werden oder alternative Phosphoramidit-Derivate zum Einsatz kommen. Anstelle von MMTr können auch andere geeignete Schutzgruppen eingesetzt werden, wie z.B. Trityl, Dimethoxytrityl und Pixyl (9-(9-Phenyl)xanthenyl).

Die Verbindungen der Formel VI können nach den in J. March (Advanced Organic Chemistry, 3rd. Ed, J. Wiley, New York 1985) und S. Agrawal, Ed. (Protocols for Oligonucleotides and Analogs, Methods in Molecular Biology, Vol. 20, Humana Press 1993) beschriebenen Verfahren hergestellt werden.

Antisense-Oligonucleotide der Formel V werden dadurch erhalten, daß man zunächst ein Oligonucleotid mit einem 3'-Aminospacer nach bekannten Verfahren synthetisiert und anschließend mit einer Verbindung der Formel VII 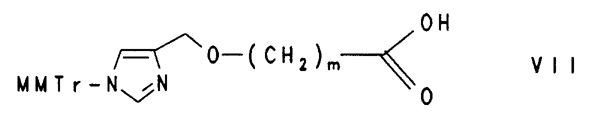
worin
MMTr für Monomethoxytrityl steht,
nach bekannten Verfahren, wie beispielsweise in S. Agrawal (Ed. Protocols for Oligonucleotides and Analogs, Methods in Molecular Biology, Vol. 20, Humana Press 1993) beschrieben, umsetzt und anschließend die MMTr-Schutzgruppe abspaltet, bevorzugt wird die MMTr-Gruppe durch Behandlung mit Essigsäure abgespalten.
Alternativ zu MMTr können auch andere geeignete Schutzgruppen, wie z.B. Trityl, Dimethoxytrityl und Pixyl eingesetzt werden.

Gegenstand der Erfindung ist weiterhin ein Verfahren zur Herstellung eines therapeutischen Mittels, das dadurch gekennzeichnet ist, daß man die erfindungsgemäßen Oligonucleotide mit einem physiologisch annehmbaren Träger sowie gegebenenfalls weiteren Zusatz- und/oder Hilfsstoffen in eine geeignete Zubereitungsform bringt.

Die therapeutischen Mittel der vorliegenden Erfindung können beispielsweise verwendet werden zur Behandlung von Erkrankungen, die durch Viren hervorgerufen werden, beispielsweise durch HIV, HSV-1, HSV-2, Influenza, VSV, Hepatitis B oder Papilloma Viren, oder zur Behandlung von Krebs, wobei dabei beispielsweise Oligonucleotid-Sequenzen zum Einsatz kommen können, die gegen Targets gerichtet sind, die für Krebsentstehung bzw. Krebswachstum verantwortlich sind. Solche Targets sind beispielsweise:
1) Nucleare Onkoproteine wie beispielsweise c-myc, N-myc, c-myb, c-fos, c-fos/jun, PCNA, p120
2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl
3) Zelluläre Rezeptoren wie beispielsweise EGF-Rezeptor, c-erbA, Retinoid-Rezeptoren, Protein-Kinase regulatorische Untereinheit, c-fms
4) Cytokine, Wachstumsfaktoren, Extrazelluläre Matrix-Proteine wie beispielsweise CSF-1, IL-6, IL-1a, IL-1b, IL-2, IL-4, bFGF, Myeloblastin, Fibronectin,
   Ferner eignen sich die therapeutischen Mittel der vorliegenden Erfindung beispielsweise zur Behandlung von Erkrankungen, die durch Integrine oder Zell-Zell-Adhäsionsrezeptoren beeinflußt werden, beispielsweise durch VLA-4, VLA-2, ICAM oder ELAM.

Die therapeutischen Mittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Sie können auch rektal, z.B. in Form von Suppositorien, oder parenteral, z.B. in Form von Injektionslösungen, verabreicht werden. Für die Herstellung von pharmazeutischen Präparaten können die Oligonucleotide in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

Eine bevorzugte Form der Verabreichung ist die Injektion. Hierzu werden die erfindungsgemäßen Oligonucleotide in einer flüssigen Lösung, vorzugsweise in einem physiologisch annehmbaren Puffer, wie z.B. Hank's Lösung oder Ringer's Lösung, formuliert. Die erfindungsgemäßen Oligonucleotide können aber auch in fester Form formuliert werden und vor dem Gebrauch gelöst oder suspendiert werden. Die für die systemische Verabreichung bevorzugten Dosierungen betragen ca. 0,01 mg/kg bis ca. 50 mg/kg Körpergewicht und Tag.

### Beispiele:

1) 1-(6-[5'-Methyl-3'-(4''-methoxytriphenylmethyl)imidazol]ether)-hexyl-O-phosphorigsäure-β-cyano-ethylester-N,N-diisopropylamid
   1a. 1-tert.-Butyldimethylsiloxy-6-bromhexan
      5g (27,8 mmol) 6-Brom-1-hexanol wurden in 10 ml absol. Dimethylformamid (DMF) gelöst, mit 4,73 g (69 mmol) Imidazol und 5,02g (33 mmol) tert.-Butyldimethylchlorsilan versetzt und 10 h bei 35 °C gerührt. Es wurden 50 ml Wasser zugegeben und 30 min bei Raumtemperatur gerührt. Danach wurde das Reaktionsgemisch zwischen Wasser und Dichlormethan (DCM) verteilt, die organische Phase mit Na₂SO₄ getrocknet und das Lösungsmittel im Vakuum eingedampft. Das Produkt wurde durch Chromatographie an Kieselgel (n-Heptan/Essigester (EE) 1:1) gereinigt.
      Ausbeute: 5,02 g (62%);
      ¹H-NMR (DMSO, 200 MHz): 0,02 (s, 6H, Si-CH₃); 0,86 (s, 9H, Si-tBu); 1,21-1,57 (m, 4H, (CH₂)₂); 1,60-1,85 (m, 2H, CH₂); 3,48-3,70 (m, 4H, CH₂-Br & CH₂-O)
   1b. 3-(4'-Methoxy-triphenylmethyl)-5-hydroxymethylimidazol
      3g (22,3 mmol) 5-Hydroxymethylimidazolhydrochlorid wurden zweimal mit absol. Acetonitril koevaporiert, dann in 100 ml absol. Pyridin suspendiert. Unter Eiskühlung wurden 7,27 g (67 mmol) Trimethylchlorsilan zugegeben. Es wurde 30 min bei Raumtemperatur (RT) gerührt, anschließend 10,34 g (33,5 mmol) 4-Methoxytriphenylchlormethan zugegeben und 3h bei RT gerührt. Unter Eiskühlung wurden zunächst 25 ml Wasser, dann 50 ml konz. Ammoniak zugetropft, 30 min bei RT gerührt, dann das Lösungsmittel im Vakuum abdestilliert. Der Rückstand wurde zwischen Wasser und DCM verteilt, die organische Phase mit Na₂SO₄ getrocknet, das Lösungsmittel im Vakuum eingedampft. Der Rückstand wurde mit Ether verrührt, abgesaugt und mit Ether nachgewaschen.
      Ausbeute: 6,33 g (77%);
      MS (FAB): m/e = 377.3 (M + Li⁺, 30%);
      ¹H-NMR (DMSO, 200 MHz): 3,77 (s, 3H, OCH₃); 4,33 (d, 2H, CH₂-O); 4,85 (t, 1H, OH); 6,64-7,50 (m, 16H, Ar-H, Imidazol-H).
   1c. 1-tert.-Butyldimethylsiloxy-6-(5'-methyl-3'-(4''-methoxytriphenylmethyl)imidazol)ether)hexan
      129,3 mg (2,9 mmol) Natriumhydrid (55-65% ölige Dispersion) wurden mit 10 ml n-Heptan gewaschen und in 15 ml absol. DMF suspendiert. Dann wurde 1 g (2,7 mmol) 3-(4'-Methoxy-triphenylmethyl)-5-hydroxymethylimidazol (Beispiel 1b) zugegeben und 1h bei RT gerührt. Danach wurden innerhalb von 10 min 0,877 g (2,9 mmol) 1-tert.-Butyldimethylsiloxy-6-bromhexan (Beispiel 1a), gelöst in 10 ml absol. DMF zugetropft und 12 h bei RT gerührt. Danach wurden 50 ml Wasser zugegeben, 10 min bei RT gerührt, das Reaktionsgemisch zwischen Wasser und Essigsäureethylester (EE) verteilt, die organische Phase mit Na₂SO₄ getrocknet, das Lösungsmittel im Vakuum eingedampft. Das Produkt wurde durch Chromatographie an Kieselgel (DCM/EE/Triethylamin (TEA) 90:9:1) gereinigt.
      Ausbeute: 1200 mg (70%);
      MS (FAB) M/e = 591.5 (M + Li⁺, 30%); ¹H-NMR (DMSO, 200 MHz): 0,01 (s, 6H, Si-CH₃); 0,83 (s, 9H, Si-tBu); 1,19-1,35 (m, 4H, (CH₂)₃); 1,36-1,54 (m, 4H, (CH₂)₂); 3,35 (t, 2H, CH₂-O); 3,53 (t, 2H, CH₂-Br); 3,76 (s, 3H, OCH₃); 4,25 (s, 2H, CH₂-O); 6,75-7,48 (m, 16H, Ar-H, Imidazol-H).
   1d. 1-[6-(5'-Methyl-3'-(4''-methoxytriphenylmethyl)imidazol)ether)]hexanol
      1200 mg (2,1 mmol) 1-tert.-Butyldimethylsiloxy-6-(5'-methyl-3'-(4''-methoxytriphenylmethyl)imidazol)ether)hexan (aus Beispiel 1c) wurden in 5ml absol. Tetrahydrofuran (THF) und 3 ml absol. DMF gelöst und bei 0 °C mit einer Lösung von 3,73 ml (4,1 mmol) Tetrabutylammoniumfluorid (1,1M in absol. THF) versetzt und 2h bei RT gerührt. Das Reaktionsgemisch wurde eingedampft und durch Chromatographie an Kieselgel (DCM/EE/Triethylamin (TEA) 95:4:1) gereinigt.
      Ausbeute: 100%
      MS (FAB): m/e = 477.3 (M + Li⁺);
      ¹H-NMR (DMSO, 200 MHz):1,15-1,58 (m, 8H, (CH₂)₄); 3,25-3,42 (m, 4H, CH₂-O & CH₂-OH); 3,78 (s, 3H, OCH₃); 4,21-4,36 (m, 3H, Imidazol-CH₂-O & OH); 6,75-7,49 (m, 16H, Ar-H, Imidazol-H).
   1e. 1-(6-[5'-Methyl-3'-(4''-methoxytriphenylmethyl)imidazol]ether)-hexyl-O-phosphorigsäure-β-cyano-ethylester-N,N-diisopropylamid
      1,0528 g (2,24 mmol) 1-[6-(5'-Methyl-3'-(4''-methoxytriphenylmethyl)imidazol)ether)]hexanol (aus Beispiel 1d) wurden in 7 ml absol. THF gelöst und unter Schutzgas mit 778 ml (4,47 mmol) Diisopropylethylamin (DIPEA) versetzt. Innerhalb von 5 min wurde eine Lösung von 635,4 mg (2,68 mmol) 2-Cyanoethyl-N,N-diisopropyl-chlorophosphoramidit in 3 ml absol. THF zugetropft und 2 h bei RT gerührt. Der Niederschlag wurde abgesaugt, das Filtrat mit EE verdünnt und die organische Phase zweimal mit 0.1M NaH₂PO₄-Puffer (pH 7) extrahiert. Die organische Phase wurde mit Na₂SO₄ getrocknet und das Lösungsmittel im Vakuum eingedampft. Das Produkt wurde durch Chromatographie an Kieselgel (EE/TEA 99:1) gereinigt.
   Ausbeute: 933 mg (62%);
   MS (FAB) m/e = 693.4 (M + Na⁺); 677.4 (M + Li⁺);
   ¹H-NMR (DMSO, 200 MHz):1,04-1,60 (m, 20H,CH(CH₃)₂ & (CH₂)₄); 2,75 (t, 2H, CH₂-CN); 3,36 (t, CH₂-O-CH₂-Imi); 3,42-3,77 (m, 6H, CH₂-O-P & CH₂-CH₂-CN & CH(CH₃)₂); 3,79 (s, 3H, OCH₃); 4,225 (s, 2H, Imidazol-CH₂-); 6,75-7,48 (m, 16H, Ar-H, Imidazol-H). ³¹P-NMR (DMSO): 147.1.
2) 6-[5'-Methyl-3'-(4''-methoxytriphenylmethyl)imidazolether]-hexansäure
   2a. 6-[5'-Methyl-3'-(4''-methoxytriphenylmethyl)imidazolether]-hexanal
      1,15 mg (9,1 mmol) Oxalylchlorid wurden in 50 ml absol. DCM gelöst und bei -78 °C 1,484 g (19 mmol) Dimethylsulfoxid (DMSO) innerhalb von 5 min zugetropft. Dann wurden 1,86g 1-[6-(5'-Methyl-3'-(4''-methoxytriphenylmethyl)imidazol)ether)]hexanol (aus Beispiel 1d), gelöst in 10 ml absol. DCM innerhalb von 15 min zugetropft, wobei die Temperatur nicht über -60 °C anstieg. Es wurden 1,5 h bei -78 °C gerührt, dann 11 ml TEA zugetropft, wobei die Temperatur nicht über -60 °C anstieg. Es wurde auf RT erwärmt und mit Wasser extrahiert und die wässrige Phase noch zweimal mit DCM nachgewaschen. Die organischen Phasen wurden vereinigt und dreimal mit gesättigter NaCl-Lösung gewaschen, mit Na₂SO₄ getrocknet und das Lösungsmittel im Vakuum eingedampft. Das Produkt wurde an Kieselgel chromatographiert (EE/TEA 99:1).
      Ausbeute: 1034 mg;
      MS (FAB): 507.3 (M + Na⁺); 475.3 (M + Li⁺);
      ¹H-NMR (DMSO, 200 MHz):1,19-1,60 (m, 8H, (CH₂)₄); 2,40 (dt, 2H, CH₂-CHO); 3,36 (t, 2H, CH₂-O); 3,78 (s, 3H, OCH₃); 4,25 (s, Imidazol-CH₂-); 6,78-7,50 (m, 16H, Ar-H, Imidazol-H); 9,63 (t, 1H, CHO).
   2b. 6-[5'-Methyl-3'-(4''-methoxytriphenylmethyl)imidazolether]-hexansäure
      92,7g (0,54 mmol) Silbernitrat wurden in 400 ml Wasser gelöst und zu einer Lösung von 100 mg (0,21 mmol) 6-[5'-Methyl-3'-(4''-methoxytriphenylmethyl) imidazolether]-hexanal (aus Beispiel 2a) in 3,6 ml Ethanol getropft. Anschließend wurde eine Lösung von 255 mg (4,54 mmol) KOH in 4,2 ml Wasser zugegeben und 2h bei RT gerührt. Der Niederschlag wurde abfiltriert und mit Wasser und Methanol gewaschen. Anschließend wurde das Filtrat mit 3,5% Oxalsäurelösung bis pH5 angesäuert. Es wurde zwischen Wasser und DCM verteilt, die organische Phase mit Na₂SO₄ getrocknet und das Lösungsmittel im Vakuum eingedampft.

   Ausbeute: 115 mg (92%);
   MS (FAB): m/e = 485.2 (M + H⁺).
3) O-N-Succinimidylester der 6-[5'-Methyl-3'-(4''-methoxytriphenylmethyl)imidazolether]-hexansäure
   20 mg (34,2 mmol) 6-[5'-Methyl-3'-(4''-methoxytriphenylmethyl)-imidazolether]-hexansäure aus Beispiel 2 in 1 ml absol. DMF wurden mit 6,4 ml (37 mmol) Diisopropylethylamin (DIPEA) und 12,3 mg (37,6 mmol) TSTU (O-(N-Succinimidyl)-N,N,N',N'-tetramethyluronium-tetrafluoroborat) 5h inkubiert. Danach wurden weitere 3 mg TSTU und 2 ml DIPEA zugegeben. Das Reaktionsgemisch wurde eingedampft, der Rückstand in Wasser suspendiert und mit Chloroform extrahiert. Anschließend wurde die organische Phase eingedampft.
   Ausbeute: 20,6 mg (90%)
4) Oligonucleotidsynthese
   Unmodifizierte Oligonucleotide wurden auf einem automatischen DNA Synthesizer (Applied Biosystems Model 380B oder 394) unter Anwendung der Standard Phosphoramidit-Chemie und Oxidation mit Jod synthetisiert. Zur Einführung von Phosphorothioat-Brücken in gemischten Phosphorothioaten und Phosphodiester Oligonucleotiden wurde anstelle von Jod mit TETD (Tetraethylthiuramdisulfid) oxidiert (Applied Biosystems User Bulletin 65). Nach Abspaltung vom festen Träger (CPG oder Tentagel) und Entfernung der Schutzgruppen mit konz. NH₃ bei 55 °C während 18h, wurden die Oligonucleotide zunächst durch Butanol-Fällung (Sawadogo, Van Dyke, Nucl. Acids Res. 19 (1991) 674 ) gereinigt. Anschließend wurde das Natriumsalz erhalten durch Ausfällung aus einer 0,5 M NaCl Lösung mit 2,5 Volumenteilen Ethanol.
   Die Analyse der Oligonucleotide erfolgte durch
   a) Analytische Gelelektrophorese in 20% Acrylamid, 8M Harnstoff, 454M Tris-borat Puffer, pH 7.0 und/oder
   b) HPLC-Analyse: Waters GenPak FAX, Gradient CH₃CN (400 ml), H₂O (1.6 l), NaH₂PO₄ (3.1 g), NaCl (11.7g), pH 6.8 (0.1M an NaCl) nach CH₃CN (400 ml), H₂O (1.6 l), NaH₂PO₄ (3.1 g), NaCl (175.3g), pH 6.8 (1.5 M an NaCl) und/oder
   c) Kapillargelelektrophorese Beckmann Kapillare eCAP™, U100P Gel Column, 65 cm length, 100 mm I.D., window 15cm from one end, Puffer 140 mM Tris, 360 mM Borsäure, 7M Harnstoff und/oder
   d) Elektrospray Massenspektroskopie.

   Die Analyse der Oligonucleotide ergab, daß diese jeweils in einer Reinheit von größer 90% vorliegen.
   Folgende Oligonucleotide wurden synthetisiert:
   4a. [5'-Imidazol]--G*C*A G G A G G A T G C T G A G G A* G*G -3' 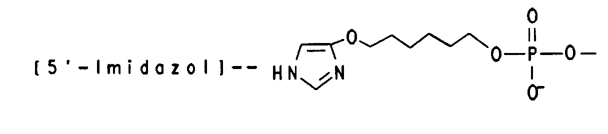 Zur Einführung des [5'-Imidazol]-Restes wurde zunächst das Oligonucleotid an der festen Phase synthetisiert, dann die 5'-Dimethoxytritylgruppe abgespalten. Anschließend wurde mit 1-(6-[5'-Methyl-3'-(4''-methoxytriphenylmethyl) imidazol]ether)-hexyl-O-phosphorigsäure-β-cyano-ethylester-N,N-diisopropylamid (aus Beispiel 1) und Tetrazol umgesetzt, dann mit Jod oxidiert und die Monomethoxytritylschutzgruppe abgespalten, wobei nach dem Standard Phosphoramidit-Protokoll (Applied Biosystems Model 392/394, USER MANUAL, June 1992) vorgegangen wurde. Es wurde lediglich ein Standard-Nucleosidphosphoramidit durch 1-(6-[5'-Methyl-3'-(4''-methoxytriphenylmethyl) imidazol]ether)-hexyl-O-phosphorigsäure-β-cyano-ethylester-N,N-diisopropylamid ersetzt.
   4b. [5'-Imidazol]--A*C*G T T C C T C C T G C G G G A A*G*G -3'
   4c. [5'-Imidazol]--G*C*G G G G C T C C A T G G G G G T*C*G -3'
   4d. [5'-Imidazol]--C G C T T G T G G A -3'
   4e. 5'- G G A C C G A A G G--[3'-Imidazol] 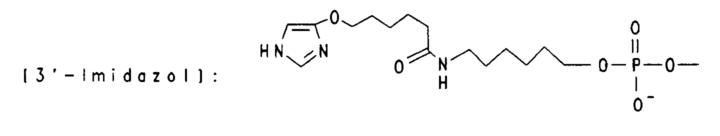 Zur Einführung des [3'-Imidazolrestes] wurde zunächst das Oligonucleotid mit dem 3'-Phosphorsäure-(6-aminohexyl)ester Rest wie in C. R. Petrie et al. (Bioconj. Chem. 3 (1992) 85) beschrieben synthetisiert. Anschließend wurde mit dem O-N-Succinimidylester aus Beispiel 3 umgesetzt. Dazu wurden 20 OD des Oligonucleotids in 500 ml mit 7 mg des -O-N-Succinimidylesters und 500 ml DMF und 100 ml Puffer (1M NaHCO₃ und 1M Na₂CO₃, pH 9) versetzt und über Nacht bei RT inkubiert. Die Reinigung erfolgte über eine ®Sephadex G25M PD10 Säule (Fa. Pharmacia). Es wurde eingedampft und die Monomethoxytritylschutzgruppe durch Inkubation mit 500 ml Essigsäure (80%) bei RT für 1h abgespalten. Anschließend wurde erneut eingedampft, der Rückstand in 1 ml Wasser gelöst und mit 0,5 ml Chloroform extrahiert. Das Produkt wurde mit 0,5M Ammoniumchlorid und Ethanol/i-Propanol gefällt.
5) RNA-Synthese und Spaltung: Folgende RNA-Sequenz: 
r-(GGA GCC CAG CUA UGA ACU CCU UCU CCA CAA GCG CCU UCG GUC
CAG UUG CCU UCU CCC UGG GGC)
 wurde nach der Vorschrift von J. F. Milligan et al. (Methods Enzymol 180 (1989) 51) hergestellt. Sie ist an der unterstrichenen (AO-2) bzw. doppelt unterstrichenen (AO-1) Stelle gegen die Imidazol-derivatisierten Oligonucleotide aus Beispiel 4e bzw. 4d komplementär. Erste Ergebnisse zeigen, daß die RNA durch Inkubation mit den beiden Oligonucleotiden effektiv gespalten wird, während Inkubation mit jeweils nur einem Oligonucleotid unter gleichen Bedingungen nicht zur Spaltung führt.

## Patentansprüche

1. Therapeutische Mittel zur Inhibierung der Protein-Synthese enthaltend zwei Antisense-Oligonucleotide AO-1 und AO-2 mit ca. 6 - 100 Nucleotiden, die an benachbarter Stelle an der Ziel-RNA binden und am 5'-Ende bzw. 3'-Ende über geeignete Spacer mit Konjugatmolekülen A und B verknüpft sind, wobei die Konjugatmoleküle A und B jedes für sich nicht reaktiv ist, aber in räumliche Nähe gebracht, eine Einheit bilden, die RNA katalytisch zu spalten vermag oder durch die räumliche Nähe aktiviert zur kovalenten Bindung von RNA führt.

2. Therapeutisches Mittel gemäß Anspruch 1, dadurch gekennzeichnet, daß die Antisense Oligonucleotide aus 10 - 40 Nucleotiden bestehen.

3. Therapeutisches Mittel gemäß Anspruch 1 oder 2, dadurch gekennzeichnet, daß nachfolgende Konjugatmoleküle A und B eingesetzt werden:
a) A = B = Imidazol;
b) A = Imidazol, B = Molekül der Formel I 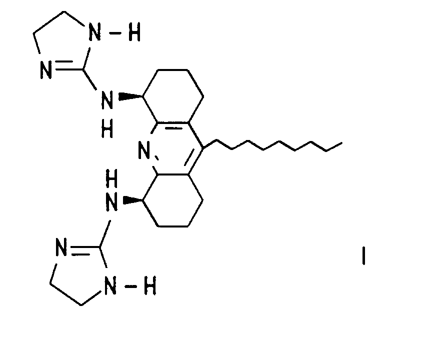
c) A = B = 1,10-Phenanthrolin;
d) A = L-Lysin, B = L-Tryptophanyl-L-Lysin;
e) A = L-Lysyl-L-Tryptophan, B = L-Lysin;
f) A = Glycin, B = Glycyl-L-Histidin;
g) A = Glycyl-Glycin, B = L-Histidin;
h) A = Glycin, B = L-Histidyl-L-Lysin;
i) A = Glycyl-L-Histidin, B = L-Lysin;
wobei die Konjugatmoleküle aus c) und f) - i) jeweils mit dem Oligonucleotid über einen geeigneten Spacer zur Bildung eines Cu(II)-Komplexes verknüpft sind.

4. Therapeutische Mittel gemäß den Ansprüchen 1 bis 3, dadurch gekennzeichnet, daß die Antisense-Oligonucleotide unmodifiziert oder modifiziert vorliegen.

5. Verfahren zur Spaltung von RNA bzw. Crosslinking, dadurch gekennzeichnet, daß man zwei Antisense-Oligonucleotide AO-1 und AO-2 mit ca. 6 - 100 Nucleotiden, die an benachbarter Stelle an der Ziel-RNA binden und am 5'-Ende bzw. 3'-Ende über geeignete Spacer mit Konjugatmolekülen A und B verknüpft sind, wobei die Konjugatmoleküle A und B jedes für sich nicht reaktiv ist, in räumliche Nähe bringt, so daß diese eine Einheit bilden und die RNA katalytisch spalten oder aktiviert werden und so zur kovalenten Bindung von RNA führen.

6. Antisense-Oligonucleotide AO-1 und AO-2 bestehend aus ca. 6 - 100 Nucleotiden, die am 5'-Ende bzw. am 3'-Ende über geeignete Spacer mit Konjugatmolekülen A und B verknüpft sind.

7. Verfahren zur Herstellung der Antisense-Oligonucleotide AO-1 und AO-2 gemäß Anspruch 6, dadurch gekennzeichnet, daß man jedes der Konjugatmoleküle A und B über einen geeigneten Spacer mit Oligonucleotiden verknüpft.

8. Verfahren zur Herstellung eines therapeutischen Mittels gemäß den Ansprüchen 1 bis 4, dadurch gekennzeichnet, daß man die Oligonucleotide mit einem physiologisch annehmbaren Träger sowie gegebenenfalls weiteren Zusatz- und/oder Hilfsstoffen in eine geeignete Zubereitungsform bringt.
